(19) Europäisches Patentamt
European Patent Office
Office européen des brevets

(11) **EP 2 459 161 B1**

(12) **FASCICULE DE BREVET EUROPEEN**

(45) Date de publication et mention de la délivrance du brevet:
**28.03.2018 Bulletin 2018/13**

(21) Numéro de dépôt: **10734762.7**

(22) Date de dépôt: **27.07.2010**

(51) Int Cl.:
*A61K 8/60* (2006.01)      *A61K 8/64* (2006.01)
*A61K 8/97* (2017.01)      *A61Q 19/00* (2006.01)

(86) Numéro de dépôt international:
**PCT/EP2010/060876**

(87) Numéro de publication internationale:
**WO 2011/012615 (03.02.2011 Gazette 2011/05)**

(54) **COMPOSITION COSMETIQUE POUR LE TRAITEMENT DE L'ACNE COMPRENANT UN EXTRAIT PEPTIDIQUE DE SCHIZANDRA SPHENANTHERA**

KOSMETISCHE ZUSAMMENSETZUNG ZUR BEHANDLUNG VON AKNE MIT EINEM SCHIZANDRA SPHENANTHERA-PEPTIDEXTRAKT

COSMETIC COMPOSITION FOR THE TREATMENT OF ACNE COMPRISING A PEPTIDE EXTRACT OF SCHIZANDRA SPHENANTHERA

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **30.07.2009 FR 0955343**

(43) Date de publication de la demande:
**06.06.2012 Bulletin 2012/23**

(73) Titulaire: **Laboratoires Expanscience**
**92048 Paris La Défense Cedex (FR)**

(72) Inventeurs:
• **GARNIER, Sébastien**
**06650 Le Rouret (FR)**
• **NAAIMI, Dalale**
**69150 Decines Charpieu (FR)**
• **BAUDOUIN, Caroline**
**78120 Rambouillet (FR)**

(74) Mandataire: **Regimbeau**
**87 rue de Sèze**
**69477 Lyon Cedex 06 (FR)**

(56) Documents cités:
**FR-A1- 2 910 815     KR-A- 20020 001 931**

• **DATABASE WPI Week 200821 Thomson Scientific, London, GB; AN 2008-C80978 XP002579998, & CN 101 040 971 A (WANG A) 26 septembre 2007 (2007-09-26) cité dans la demande**

## Description

## INTRODUCTION

L'invention se rapporte à une composition cosmétique pour son utilisation dans le traitement ou la prévention de l'acné comprenant un extrait peptidique de *Schizandra* et un excipient approprié.

Il existe dans le monde à peu près 25 espèces appartenant au genre *Schizandra*. Environ seize d'entre elles sont chinoises. Ces arbustes sont originaires du Nord de la Chine et des régions adjacentes de Russie et Corée.

*Schizandra* est une plante dioïque (pieds aux fleurs mâles et femelles distincts). Le fruit se présente sous forme de grappe pendante qui rappelle un peu celle du groseillier. Elle est pourvue d'un pédoncule nu dans sa partie supérieure (environ 5-10 cm) qui est recouvert dans sa partie inférieure par des baies rouges vifs, un peu plus grosses, plus compactes et plus fermes que la groseille. La graine, sphérique, mesure quelques millimètres.

Deux espèces sont officiellement reconnues comme médicinales en Chine, *S. chinensis* et *S. sphenanthera* [He et al., 1997]. Leurs baies sont utilisées en médecine traditionnelle chinoise pour le traitement de la toux, de l'asthme, des sueurs nocturnes, des éjaculations nocturnes et de la diarrhée chronique. Elles sont aussi utilisées comme tonifiants et pour le traitement de la fatigue chronique.

De nom botanique *Schizandra,* cette plante appartient à la classe des *Magnoliopsida* et à l'ordre des *Magnoliales.* La famille botanique est celle des *Schisandracea.* Le terme *Schizandra* utilisé tel quel peut désigner les deux plantes différentes que sont *Schizandra chinensis* et *Schizandra sphenanthera.* Longtemps ces deux espèces ont été considérées comme équivalentes, elles pouvaient être désignées selon leur origine « Northern Schizandra » pour *Schizandra chinensis* et « Southern *Schizandra* » pour *Schizandra sphenanthera.*

*Schizandra chinensis* est aussi appelée pinyin, wǔ wèi zi, littéralement "baie à 5 parfums ». *Schizandra chinensis* est une liane arborescente caduque sauvage à croissance lente pouvant atteindre 9 à 10 mètres de hauteur.

*Schizandra sphenanthera* Rehd. et Wils. est aussi appelée : schisandre à fleurs orangées (en France), southern *Schizandra,* lemon wood (en Angleterre), hua zhong wu wei zi, nan wu wie zi (en Chine).

Si le fruit de *S. Chinensis* a été l'objet d'une littérature conséquente, c'est beaucoup moins le cas de *S. sphenanthera.* Plusieurs motifs expliquent ceci :

- une moindre réputation quant à son utilisation selon la tradition chinoise,
- une quasi-absence d'usage en occident,
- une moindre teneur en néo-lignanes totaux comparée à *S. chinensis.*

L'une des différences majeures entre les deux espèces porte sur la nature et les teneurs des néo-lignanes contenus dans leurs fruits respectifs. Cette sous-famille de constituants chimiques représente la grande originalité du genre *Schizandra.* Récemment, l'article (Huyke et al., 2007) a rapporté une étude comparative des effets sur la prolifération cellulaire d'extraits de *S. chinensis* et *S. sphenanthera.*

En Europe, aucun médicament autorisé par les Autorités Compétentes ne contient d'extrait de *Schizandra sphenanthera.* Il est généralement considéré que *S. sphenanthera* est inférieur à *S. chinensis* au plan médicinal et qu'il n'est utile qu'en tant que source alternative des lignanes actifs.

Sur un plan commercial, le fruit de *S. sphenanthera* est considéré comme moins coûteux que celui de *S. chinensis.*

## Caractéristiques des fruits

Le fruit sec de *Schizandra* comprend environ 20% d'huiles essentielles dont 7 à 30 % d'insaponifiables (Huyke et al., 2007). Les lignanes sont compris dans la fraction insaponifiable des huiles. D'autres constituants actifs sont les phytostérols et les vitamines C et E.

L'huile essentielle de *Schizandra* est riche en dérivés sesquiterpéniques [Song et al., 2007] tels que : δ-cadinène (25,6 %), γ-cadinène, β-himachalène, santalol [Huyke et al., 2007]. L'huile essentielle du fruit de *S. chinensis* contient davantage d'hydrocarbures monoterpéniques que celle de *S. sphenanthera* [Huyke et al., 2007].

Le fruit de *S. sphenanthera* se caractérise par sa richesse en désoxyschisandrine. Par contre, ses teneurs en schisandrine et γ-schisandrine sont très faibles comparées à celles de la graine de *S. chinensis* (Zhu et al., 2007).

## Les extraits de fruit : **formes utilisées**

La forme la plus utilisée en médecine traditionnelle est le fruit séché. A côté de son emploi traditionnel en Chine à l'état déshydraté, le fruit de *Schizandra* est aussi utilisé sous forme d'extraits obtenus par des solvants d'extraction permettant d'entraîner les néo-lignanes du fruit. Les solvants mentionnés dans la littérature sont l'éthanol, le $CO_2$-su-

percritique ou $CO_2$-SC (associé ou pas avec un co-solvant) et l'hexane.

Plusieurs études se sont attachées à comparer les pouvoirs extractifs du $CO_2$-SC, du chloroforme, du méthanol et de l'éthanol à l'égard des néo-lignanes du fruit.

Selon une publication récente, l'extraction du fruit de *Schizandra* par le $CO_2$ ou le $CO_2$ + 5 % d'éthanol, ou encore par l'hexane, aboutit à des compositions d'extraits assez similaires au plan néo-lignanes. Par contre, l'utilisation d'éthanol a conduit à une moindre extraction de 2 néo-lignanes, la désydroschisandrine et la gomisine O, et apparemment une meilleure extraction de γ-schisandrine (Huyke et al., 2007).

Par ailleurs, une technique d'extraction a été mise au point par Zhu et al. qui a permis de comparer les quantités de lignanes contenues respectivement dans les extraits de *Schizandra chinensis* et *Schizandra sphenanthera* (Zhu et al., 2007).

## ART ANTERIEUR

De nombreuses propriétés pharmacologiques des extraits de fruits de *Schizandra* ont été rapportées dans la littérature, telles que :

- des effets de protection hépatique, connus depuis les années 1980 ;
- des effets anti-HIV ;
- une activité anti-inflammatoire et anti-tumorale ;
- une augmentation de la biodisponibilité de certains produits, lorsque ingérés en même temps (Xin HW et al., 2007).

Ainsi, de nombreux documents citent des compositions pharmacologiques qui comprennent, entre autres composés actifs, des extraits de *Schizandra* :

La demande US 2003/0026854 de Mr Zhao décrit un médicament à base de schisandrine pour traiter les maladies du foie ; la schisandrine est en particulier extraite de *Schizandra chinensis* ou *Schizandra sphenanthera.*

Les demandes WO 2006/122485 et WO 2006/1222454 de la société Guang Zhou Zhongyi Pharmaceutical décrivent des compositions destinées à traiter le diabète, comprenant des mélanges de plusieurs plantes dont *Schizandra sphenanthera.*

La demande WO 2007/020382 de la société Phynova décrit une composition comprenant des extraits de quatre plantes dont *Schizandra chinensis* ou *Schizandra sphenanthera,* pour le traitement de problèmes hépatiques, métaboliques et/ou immunitaires, et plus particulièrement destinée à traiter l'hépatite C.

La demande WO 2007/005760 décrit une composition comprenant des composés de la famille des schizandrines, gomisines, et autres composés issus d'extraits des fruits de *Schizandra chinensis* et *Schizandra sphenanthera,* pour traiter des cellules de cancer chimiorésistantes.

L'article (Huyke et al., 2007) décrit et compare les effets d'extraits de *S. sphenanthera* et *S. chinensis* sur des cellules en culture : la prolifération des cellules épidermiques de types HaCaT et A431 est inhibée de manière dose-dépendante par ces extraits, les extraits non polaires étant plus efficaces que les extraits polaires. Les auteurs de l'essai concluent que l'extrait au $CO_2$-SC de *Schizandra sphenanthera* pourrait être utile dans la prévention et le traitement des maladies inflammatoires et hyperprolifératives de la peau [Huyke et coll., 2007].

L'utilisation d'extraits de *Schizandra chinensis* dans la prévention de l'acné a été décrite dans la demande de brevet KR 2001931, dans des compositions comprenant en outre des agents d'exfoliation, choisis parmi des protéases de plantes telles que la papaïne et la bromelaïne, ou issues de micro-organismes. Ces extraits, non clairement identifiées, ne sont donc pas utilisés seuls mais en combinaison avec des agents exfoliants.

Une composition anti-acnéique de médecine traditionnelle chinoise a été décrite dans la demande CN 11040971 : elle comprend 10 parts d'acore odorant appelé «shi chang pu », 8 parts de fruit de *Schizandra,* et trois autres plantes.

Aucun de ces documents ne décrit un extrait particulier de *Schizandra,* et en particulier un extrait peptidique, ni ses effets cosmétiques et dermatologiques sur l'acné.

Les inventeurs ont maintenant découvert, d'une manière surprenante, qu'une composition comprenant un extrait peptidique de *Schizandra* permet de traiter ou de prévenir l'acné, et en particulier l'inflammation liée à la présence de la bactérie *P. acnes.*

## Physiopathologie de l'acné

L'acné est une maladie inflammatoire chronique du follicule pilosébacé qui atteint la plupart des adolescents et des adultes. La pathologie est variée dans sa présentation et sa sévérité. L'acné des adolescents est une acné moyenne ou mineure dans 85% des cas. Parmi les 15% d'acné sévère, 3 à 4% d'hommes et 0,4% de femmes ont une acné nodulaire et chronique. L'élément commun des différents tableaux réside dans la rétention sébacée qui se traduit cliniquement par des lésions élémentaires spécifiques.

La pathologie se présente sous la forme de lésions élémentaires qui sont à des stades différents d'évolutions. Elles sont rétentionnelles et/ou inflammatoires.

- Lésions rétentionnelles correspondantes à des follicules pilo-sébacés distendus ;
- Comédons fermés ou microkystes (éléments surélevés blanc de 1 à 3 mm de diamètre) ;
- Comédons ouverts ou points noirs ;
- Lésions inflammatoires superficielles (papules et pustules) et profondes (nodules) ;
- Papule : élément rouge en relief, de 1 à 5 mm de diamètre, parfois sensible, évoluant souvent vers la pustule (purulente) ;
- Nodule : plus profond, d'un diamètre supérieur à 5 mm, pouvant évoluer vers l'abcédation et la rupture.

Les lésions d'acné peuvent laisser des cicatrices atrophiques définitives, des cicatrices hypertrophiques, ou des macules érythémateuses le plus souvent transitoires et/ou pigmentées.

L'acné est une affection du follicule pilo-sébacé (follicule très particulier avec un poil atrophié) dans laquelle interviennent quatre facteurs qui sont étroitement imbriqués et se succèdent : une hyperséborrhée, une hyperkératinisation, la prolifération d'un germe : le *Probionibacterium acnes* (*P. acnes*) et une inflammation.

**L'hyperséborrhée**

L'hyperséborrhée (=hypersécrétion sébacée) est le facteur initial de l'acné. Elle est essentiellement hormono-dépendante, en particulier sous l'effet des androgènes. En effet, la sécrétion du sébum est déclenchée et entretenue par la dihydrotestostérone (DHT) produite dans les cellules sébacées par la 5 $\alpha$-réductase de type I à partir de la testostérone libre. Au cours de l'acné les androgènes circulants sont présents à des taux normaux et la pathologie résulte d'une sensibilité particulière de la glande sébacée aux androgènes en partie due à une hyperactivité locale de la 5 $\alpha$-réductase.

Le sébum est constitué d'un mélange de squalène (12%), de cires (26%), de cholestérol, d'esters de cholestérol et de triglycérides. Le sébum des peaux acnéiques contient une concentration d'acides gras et d'oxydes de squalène (pro-inflammatoire) en quantité plus abondante par rapport aux sujets non acnéiques, faisant suite à l'hydrolyse des triglycérides par la lipase secrétée par *P. acnes.* Parallèlement, il existe un déficit en acide linoléique (anti-inflammatoire) dans les comédons comparativement à la surface épidermique sous-jacente.

Cette inversion de la balance entre lipides pro-inflammatoires et anti-inflammatoires serait à l'origine des facteurs étiopathogéniques de l'acné, à savoir le trouble de la kératinisation de l'épithélium folliculaire qui prélude à la formation du comédon. D'une part, le squalène peroxydé active les médiateurs lipidiques de l'inflammation (augmentation du leucotriène B4 et de la prostaglandine E2) et la production de l'IL-6. D'autre part, la diminution en acide linoléique provoque l'hyperkératinisation et rend l'épiderme plus perméable et plus fragile favorisant la croissance des micro-organismes et la production de cytokines (notamment l'interleukine-1$\alpha$) et de substances chémotactiques qui induisent l'inflammation.

**Hyperkératinisation et comédogenèse**

L'hyperkératinisation correspond à une multiplication anormale des cellules qui tapissent la paroi interne du follicule pilo-sébacée (cornéocytes). Les cornéocytes desquament très rapidement et provoquent la formation du micro-comédon. L'obstruction du canal du follicule pilosébacé a lieu dans l'infra-infundibulum. Elle est due à des anomalies de la prolifération, de l'adhésion et de la différenciation des kératinocytes qui ne se détachent pas les uns des autres et obstruent la lumière du canal. En temps normal, les cornéocytes se détachent laissant le sébum passer librement au niveau des pores. Dans le cas de l'hyperkératinisation, les cornéocytes restent attachés les uns aux autres, ce qui entrave la sortie du sébum. Ainsi, non seulement le sébum est sur-sécrété mais son écoulement est freiné par le rétrécissement des pores qui finissent par se boucher. Le flux de sébum bloqué à l'intérieur du follicule pilo-sébacé ainsi que les débris de cellules forment « des bouchons cornés » durs et légèrement blancs qu'on appelle "microkystes" ou comédons fermés.

Hyperprolifération kératinocytaire : Les kératinocytes du canal du follicule pilo-sébacé ont un indice de prolifération supérieur (marquage Ki67) chez le sujet acnéique comparé au sujet sain témoin. Cette augmentation de l'indice de prolifération est retrouvée en zones lésionnelles et en zones acnéiques apparemment saines. Elle est liée notamment à la production de l'IL-1$\alpha$ qui est responsable des caractéristiques du micro-comédon (hyperprolifération et différenciation anormale).

Rôle des molécules d'adhésion : Les intégrines sont des molécules d'adhésion qui assurent la cohésion entre les kératinocytes. Elles interviennent notamment dans la régulation de la prolifération et de la migration kératinocytaires. Au cours de l'acné il existe une modification de l'expression des intégrines $\alpha 2$, $\alpha 3$, $\alpha 5$ des kératinocytes de l'infra-infundibulum de follicules acnéiques. Ces modifications pourraient jouer également un rôle dans la formation du micro-comédon.

Rôle de la composition du sébum : l'hyperséborrhée diminue la concentration en acide linoléique du sébum par dilution. Cette pauvreté en acide linoléique ainsi que l'augmentation en acides gras libres induirait une anomalie de la différenciation kératinocytaire de l'infra-infundibulum intervenant dans la formation du micro-comédon mais aussi dans le déclenchement de l'inflammation.

**Prolifération bactérienne/inflammation**

Les phénomènes inflammatoires jouent un rôle fondamental dans l'acné puisqu'ils sont responsables de l'évolution de la pathologie, notamment dans la formation des lésions inflammatoires ainsi que la survenue des cicatrices.

Ce processus inflammatoire est multifactoriel et le résultat direct ou indirect de la prolifération de *P. acnes*. Cette bactérie est un germe commensal de la flore cutanée. Dans la peau normale, *P. acnes* se développe au fond du follicule pilo-sébacé et gagne la surface épidermique par le sébum. Au cours de l'acné, l'accumulation anormale de cornéocytes et l'excès de sébum dans le canal du follicule représente un environnement idéal pour son développement. Sa multiplication conduit au processus inflammatoire au cours de la pathologie.

**Mécanisme d'action de *P. acnes***

*P. acnes* produit une variété de facteurs chimiotactiques, de molécules pro-inflammatoires et de protéases qui sont responsables de la phase inflammatoire de l'acné.

D'une part, les lipases produites par *P. acnes* hydrolysent les triglycérides du sébum en acides gras libres qui sont irritants et qui libèrent des substances chimiotactiques vis-à-vis des polynucléaires. Ces polynucléaires neutrophiles libèrent ensuite dans le tissu périfolliculaire des enzymes lyzosomiales qui entraînent la rupture de la paroi du follicule pilo-sébacé avec diffusion en profondeur de l'inflammation.

D'autre part, *P. acnes* utilise l'immunité immédiate via les « toll like receptors » (récepteurs TLR de type 2 et 4) au niveau des cellules immunitaires induisant ainsi une activation brutale de la cellule avec libération de cytokines inflammatoire (IL-1$\alpha$, TNF$\alpha$, IL-6, IL-8, TGF$\alpha$). Les kératinocytes sont aussi directement impliquées par la sécrétion de cytokines, en particulier l'IL-1$\alpha$, le TNF$\alpha$ et la chemokine IL-8 après stimulation par *P. acnes.* L'augmentation de la production de l'IL-8 est corrélée à l'hyperkératinisation du follicule et est responsable de l'afflux des cellules inflammatoires au niveau du follicule pilo-sébacé (pouvoir chimio-attracteur).

L'activation des TLRs et du facteur de transcription NP$\kappa$B par *P. acnes* induit également la libération des MMPs (notamment les MMP1 ; MMP2 ; MMP3 et MMP9). Ces protéases s'attaquent aux fibres conjonctives situées au pourtour du follicule pilo-sébacé et participent à la fragilisation et la rupture du mur folliculaire distendu ainsi qu'à la diffusion de l'inflammation dans le derme. Elles vont être aussi responsables de la formation de cicatrices.

Par ailleurs, chez les patients acnéiques ayant tendance à faire des cicatrices, la réaction inflammatoire est plus intense et de durée plus longue avec une angiogenèse élevée. Dans ce sens, le VEGF, facteur de croissance des vaisseaux, joue un rôle actif dans le déclenchement de ces lésions en augmentant la perméabilité des vaisseaux sanguins qui irriguent la peau, en attirant les cellules inflammatoires/ endothéliales et en favorisant l'angiogenèse.

Enfin, *P. acnes* agit comme un super antigène activant directement les lymphocytes T en se liant à des récepteurs sans l'intervention de cellules présentatrices d'antigène, ce qui induit une activation des cellules effectrices plus rapide et plus efficace. La réaction inflammatoire se trouve ainsi amplifiée. En définitif, l'extension de la réaction inflammatoire rend compte de l'aspect clinique avec l'apparition de papules, pustules et nodules.

**DESCRIPTION DE L'INVENTION**

Les inventeurs ont découvert que les extraits peptidiques du fruit de *Schizandra* présentent des propriétés cosmétiques et dermatologiques jamais décrites jusqu'à présent.

L'invention a pour objet une composition pour son utilisation dans le traitement ou la prévention de l'acné, comprenant un extrait peptidique et osidique de fruit de *Schizandra,* en association avec un excipient approprié, ledit extrait ne comprenant pas de lignanes et en ce que l'extrait peptidique et osidique est issu de *Schizandra sphenanthera.*

L'extrait peptidique et osidique est issu de *Schizandra sphenanthera.*

Selon un mode de réalisation préféré de l'invention, l'extrait peptidique et osidique est présent dans la composition à un pourcentage compris entre 0.01% et 15%, plus préférentiellement entre 0.1% et 5%, en poids par rapport au poids total de la composition. Selon un mode préféré de réalisation de l'invention, l'extrait peptidique et osidique est constitué de :

- 5 à 90% de peptides, et
- 5 à 90% de sucres totaux,

les pourcentages étant exprimés par rapport au poids total de la matière sèche dudit extrait peptidique.
L'extrait peptidique et osidique selon l'invention présente avantageusement les spécifications suivantes :

- 10 à 50% de peptides ;
- 10 à 60% de sucres totaux.

Dans la présente demande, les termes « extrait peptidique », « extrait peptidique et osidique » et « peptides de *Schizandra* » ont la même signification et sont utilisés indifféremment l'un de l'autre pour désigner le même extrait de fruit. L'extrait peptidique ne comprend pas de lignanes, connus jusqu'à présent pour être les principes actifs du fruit.

Selon un mode préférentiel de réalisation de l'invention, l'extrait peptidique est avantageusement obtenu par un procédé comprenant les étapes successives suivantes : à partir de fruit de *Schizandra,* une extraction par $CO_2$ supercritique permet d'obtenir une huile brute et un tourteau délipidé. Le tourteau de baies de *Schizandra,* obtenu après extraction des lipides, est dispersé dans l'eau. Il est ensuite réalisé une hydrolyse de l'amidon et des fibres (cellulose, hémicellulose, ...) à l'aide d'un mélange de cellulases et d'α-amylases, ainsi que des protéines par des protéases. Un traitement thermique permet de dénaturer les enzymes en fin de réaction. Après centrifugation, le milieu réactionnel est purifié en réalisant une ultrafiltration et une diafiltration sur une membrane ayant un seuil de coupure de 15 kDa afin d'éliminer les protéines résiduelles (retentât). Le perméat est ensuite nanofiltré afin d'éliminer des sels minéraux, puis l'extrait peptidique est décoloré avec du charbon actif puis filtré et récupéré. Enfin, le produit est filtré stérilement (0,2 μm) et peut être lyophilisé ou conditionné.

Tableau 1 : Exemple d'une composition analytique d'un extrait peptidique et osidique de *Schizandra,* en pourcentages par rapport à la matière sèche :

| Teneur en peptides | 13% |
|---|---|
| Teneur en azote alpha aminé | 6.0% |
| Teneur en sucres totaux | 44% |
| Répartition des masses molaires (en Daltons) des peptides | |
| > 3500 Da | 0% |
| 3500 - 1200 Da | 4% |
| 1200 - 300 Da | 23% |
| 300 - 130 Da | 18% |
| < 130 Da | 55% |

En fonction du type d'administration souhaitée, la composition selon l'invention comprend au moins un excipient pharmaceutiquement acceptable, notamment dermatologiquement acceptable. Si la composition est cosmétique ou dermatologique, on utilise un excipient adapté pour une administration par voie topique externe.

La composition selon la présente invention peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

La composition selon l'invention peut en outre comprendre au moins un composé anti-acnéique choisi parmi un agent sébo-régulateur, un agent anti-bactérien, un agent anti-fongique, un agent kératolytique, un agent kérato-régulateur, un agent astringent, un agent anti-inflammatoire/anti-irritant, un agent antioxydant/antiradicalaire, un agent cicatrisant, un agent anti-âge, et/ou un agent hydratant.

Par agent sébo-régulateurs on désigne par exemple les inhibiteurs de 5-alpha réductase, notamment l'actif 5-alpha Avocuta® commercialisé par les Laboratoires Expanscience. Le zinc et ses sels gluconate, salicylate et acide pyroglutamique présentent aussi une activité sébo-suppresseur. On peut citer aussi la spironolactone, anti-androgène et antagoniste de l'aldostérone, qui engendre une réduction significative du taux de sécrétion de sébum après douze semaines d'application. D'autres molécules extraites, par exemple des graines de citrouille *Cucurbita pepo,* et l'huile de pépins de courge, ainsi que le sabal limitent la production de sébum par inhibition de la transcription et de l'activité de la 5α-réductase. D'autres sébo-régulateurs d'origine lipidique agissant sur la qualité du sébum, comme l'acide linoléique, présentent un intérêt.

Par agent anti-bactérien ou antifongique, on entend des molécules limitant la croissance ou détruisant les micro-organismes pathogènes tels que certaines bactéries comme *P. acnes* ou certains champignons (*Malassezia furfur*). Les plus classiques sont les conservateurs généralement utilisés en cosmétique ou en nutraceutique, les molécules à activité anti-bactérienne (pseudo-conservateurs) tels que les dérivés capryliques (exemple : capryloyl glycine, glyceryl caprylate, ...), tels que l'hexanediol, et le sodium lévulinate, les dérivés de zinc et de cuivre (gluconate et PCA), la phytosphingosine et ses dérivés, le peroxyde de benzoyle, la piroctone olamine, le pyrithione zinc, le sulfure de sélénium, l'éconazole, le kétoconazole, ou encore les antibiotiques locaux de type érythromycine et clindamycine ...

Par agent kérato-régulateur ou kératolytique, on entend un agent qui régule ou aide l'élimination des cellules mortes de la couche cornée de l'épiderme. Parmi les agents kérato-régulateurs / kératolytiques les plus utilisés se trouvent : les acides de fruits alpha hydroxyacide - AHA (acide citrique, acide glycolique, acide malique, acide lactique...), les esters d'AHA, les associations d'AHA avec d'autres molécules comme l'association acide malique et protéines d'amandes (keratolite®), l'association acide glycolique ou acide lactique avec l'arginine ou encore l'association d'hydoxy-acide avec des molécules lipidiques comme le LHA® pour lipo-hydroxy-acide, les complexes d'hydroxyacide amphotères - AHCare, l'écorce de saule (Salix Alba bark extract), l'acide azélaïque et ses sels et esters, l'acide salicylique et ses dérivés comme l'acide capryloyl salicylique ou en association avec d'autres molécules comme l'association acide salicylique et polysaccharide (beta hydroxyacide - BHA), le tazarotène, l'adapalène, ainsi que les molécules de la famille des rétinoïdes tels que : la trétinoïne, le rétinaldéhyde, l'isotrétinoïne, le rétinol.

Par agent astringent, on entend un agent qui aide à resserrer les pores, les plus utilisés étant les polyphénols et les dérivés de zinc, l'hamamélis.

Les agents anti-inflammatoires / anti-irritants limitent la réaction inflammatoire conduite via les cytokines ou médiateurs du métabolisme de l'acide arachidonique et ont des propriétés apaisantes et anti-irritantes. Les plus classiques sont l'acide glycyrrhétinique (les dérivés de réglisse) avec ses sels et esters, l'alpha bisabolol, le ginkgo biloba, le calendula, l'acide lipoïque, le beta-carotène, la vitamine B3 (niacinamide, nicotinamide), la vitamine E, la vitamine C, la vitamine B12, les flavonoïdes (thé vert, quercétine ...), le lycopène ou la lutéine, les sucres d'Avocat, l'oléodistillat d'Avocat, l'arabinogalactane, les peptides de Lupin, un extrait total de Lupin, un extrait peptidique de Quinoa, le Cyclocéramide® (dérivé d'oxazoline), les substances anti-glycation telles que la carnosine, la n-acétyl-cystéine, les isoflavones comme par exemple la génistéine/génistine, daidzéine/daidzine, les eaux de source ou thermales (eau d'Avène, eau de la Roche Posay, eau de Saint Gervais, eau d'Uriage, eau de Gamarde), les extraits de Goji (Lycium barbarum), les peptides ou complexes d'acides aminés végétaux ou encore. la disulone topique, ou les médicaments anti-inflammatoires.

Par agent antioxydant, on entend une molécule qui diminue ou empêche l'oxydation d'autres substances chimiques. Les antioxydants/antiradicalaires pouvant être utilisés en association sont avantageusement choisis dans le groupe constitué par les thiols et les phénols, par les dérivés de réglisse comme l'acide glycyrrhétinique avec ses sels et esters, l'alpha bisabolol, l'extrait de Ginkgo biloba, de Calendula, le Cyclocéramide® (dérivé d'oxazoline), les peptides d'Avocat, les oligo-éléments comme le cuivre, le zinc, et le sélénium, l'acide lipoïque, la vitamine B12, la vitamine B3 (niacinamide, nicotinamide), les vitamines C, les vitamines E, le co-enzyme Q10, le krill, le glutathion, le butylhydroxytoluène (BHT), le butylhydroxyanisol (BHA), le lycopène ou la lutéine, le béta-carotène, la famille des polyphénols comme les tanins, les acides phénoliques, les anthocyanes, les flavonoïdes avec par exemple les extraits de thé vert, de fruits rouges, de cacao, de raisin, de *Passiflora incarnata,* de Citrus ou encore les isoflavones comme par exemple la génistéine/génistine, daidzéine/daidzine, Dans le groupe des anti-oxydants on trouve aussi les substances anti-glycation telles que la carnosine ou certains peptides, la n-acétyl-cystéine, ainsi que les enzymes antioxydants ou antiradicalaires comme la SOD (super oxyde dismutase), la catalase, la glutathion peroxydase, la thioredoxine réductase et leurs agonistes.

Les agents cicatrisants/réparateurs de la fonction barrière pouvant être utilisés en association sont avantageusement la vitamine A, le panthénol (vitamine B5), l'Avocadofurane®, les sucres d'Avocat, le lupéol, l'extrait peptidique de Maca, un extrait peptidique de Quinoa, l'arabinogalactane, l'oxyde zinc, le magnésium, le silicium, l'acide madécassique ou asiatique, le sulfate de dextran, le coenzyme Q10, la glucosamine et ses dérivés, la chondroïtine sulfate et globalement les GAG (glycosaminoglycanes), le sulfate de dextran, les céramides, le cholestérol, le squalane, les phospholipides, les peptides de Soja fermentés ou non, les peptides végétaux, les polysaccharides marins, végétaux ou biotechnologiques comme les extraits d'algues ou l'extrait de fougère, les oligo-éléments, les extraits de plantes à tanins comme les tanins dérivant de l'acide gallique appelés tanins galliques ou encore hydrolysables, initialement trouvés dans la noix de galle, et les tanins catéchiques résultant de la polymérisation d'unités flavanniques dont le modèle est fourni par le Cachou (*Acacia catechu*). Les oligo-éléments pouvant être utilisés sont avantageusement choisis dans le groupe constitué par le cuivre, le magnésium, le manganèse, le chrome, le sélénium, le silicium, le zinc et leurs mélanges.

Les agents anti-âge pouvant agir en association pour la prise en charge de l'acné sur les sujets d'âge mûr sont des agents antioxydants et en particulier la vitamine C, la vitamine A, le rétinol, le rétinal, l'acide hyaluronique de tout poids moléculaire, l'Avocadofurane®, les peptides de Lupin, l'extrait peptidique de Maca.

Les actifs hydratants / émollients les plus utilisés sont la glycérine ou ses dérivés, l'urée, l'acide pyrrolidone carboxylique et ses dérivés, l'acide hyaluronique de tous poids moléculaires, les glycosaminoglycanes et tous autres polysaccharides d'origine marine, végétale ou biotechnologique comme par exemple la gomme xanthane, le fucogel®, certains acides gras comme l'acide laurique, l'acide myristique, les acides gras mono- et poly-insaturés de type oméga 3, 6 et 7, 9 (exemple : l'acide linoléique, l'acide palmitoléique, etc), l'Oléodistillat de Tournesol, les peptides d'Avocat, le beurre de Cupuaçu.

Une association particulièrement avantageuse selon l'invention est une composition comprenant l'extrait peptidique et osidique de fruit de *Schizandra sphenanthera* et des insaponifiables végétaux et animaux, comme par exemple, les insaponifiables d'Avocat et de Soja, et des concentrats d'huile végétale ou animale en insaponifiables, comme par exemple le concentrat d'huile de Tournesol ou d'huile de Palme, ou encore des huiles végétales contenant des insaponifiables comme par exemple les huiles de Soja et de Colza, et les dérivés d'insaponifiables comme les furanes d'Avocat, les esters de stérols et les dérivés vitaminiques.

Une association particulièrement avantageuse selon l'invention est une composition comprenant l'extrait peptidique et osidique de fruit de *Schizandra sphenanthera* et des sucres d'Avocat (voir demande WO 2005/115421). Cette composition est particulièrement adaptée pour le traitement de la réparation de la barrière cutanée et de l'inflammation.

Une association particulièrement avantageuse selon l'invention est une composition comprenant l'extrait de fruit peptidique et osidique de *Schizandra sphenanthera* et des peptides d'Avocat (voir demande internationale WO 2005/105123). Cette composition est particulièrement adaptée pour le traitement de l'irritation et de l'inflammation.

Une autre association particulièrement avantageuse selon l'invention est une composition comprenant l'extrait peptidique et osidique de fruit de *Schizandra sphenanthera* et une huile d'Avocat (voir les demandes internationales WO 2004/012496, WO 2004/012752, WO 2004/016106, WO 2007/057439).

Une autre association particulièrement avantageuse selon l'invention est une composition comprenant l'extrait peptidique et osidique de fruit de *Schizandra sphenanthera* et l'Avocadofurane® (furanes d'Avocat, pouvant être obtenus par le procédé décrit dans la demande internationale WO 01/21605). Cette composition est particulièrement adaptée pour le traitement de l'inflammation, pour favoriser la cicatrisation, et pour ses propriétés anti-âge.

Une autre association particulièrement avantageuse selon l'invention est une composition comprenant l'extrait peptidique et osidique de fruit de *Schizandra sphenanthera* et les insaponifiables d'Avocat et de Soja pouvant être utilisés en association sont avantageusement un mélange d'insaponifiables d'avocat furanique et d'insaponifiables de Soja, dans un rapport respectif d'environ 1/3-2/3. Les insaponifiables d'Avocat et de Soja sont encore plus avantageusement le produit Piasclédine®, commercialisé par les Laboratoires Expanscience.

Une autre association particulièrement avantageuse selon l'invention est une composition comprenant l'extrait peptidique et osidique de fruit de *Schizandra sphenanthera* et un Oléodistillat de Tournesol, encore plus avantageusement avec un Oléodistillat de Tournesol comportant majoritairement de l'acide linoléique, tel que l'actif commercialisé par les Laboratoires Expanscience, Soline® (cf. la demande internationale WO 01/21150), Cette composition est particulièrement adaptée pour le traitement de l'inflammation et pour la réparation de la barrière cutanée.

Une autre association particulièrement avantageuse selon l'invention est une composition comprenant l'extrait peptidique et osidique de fruit de *Schizandra sphenanthera* et un insaponifiable de Soja, tel qu'obtenu selon le procédé décrit dans la demande internationale WO 01/51596.

Une autre association particulièrement avantageuse selon l'invention est une composition comprenant l'extrait peptidique et osidique de fruit de *Schizandra sphenanthera* et du Lupéol (FR 2 822 821, FR 2 857 596). Cette composition est particulièrement adaptée pour favoriser la cicatrisation.

Une autre association particulièrement avantageuse selon l'invention est une composition comprenant l'extrait peptidique et osidique de fruit de *Schizandra sphenanthera* et des peptides de Lupin tels qu'obtenus selon le procédé décrit dans la demande WO 2005/102259. Cette composition est particulièrement adaptée pour le traitement de l'inflammation et est utilisée pour ses propriétés anti-âge.

Une autre association particulièrement avantageuse selon l'invention est une composition comprenant l'extrait peptidique et osidique de fruit de *Schizandra sphenanthera* et un extrait total de Lupin (voir demande internationale WO 2005/102259). Cette composition est particulièrement adaptée pour le traitement des irritations.

Une autre association particulièrement avantageuse selon l'invention est une composition comprenant l'extrait peptidique et osidique de fruit de *Schizandra sphenanthera* et une huile de Lupin, avantageusement une huile de Lupin blanc doux, telle que celle décrite dans la demande internationale WO 98/47479.

Une autre association particulièrement avantageuse selon l'invention est une composition comprenant l'extrait peptidique et osidique de fruit de *Schizandra sphenanthera* et un extrait peptidique de Maca (voir demande internationale WO 2004/112742). Cette composition est particulièrement appréciée pour ses propriétés cicatrisantes et anti-âge.

Une association particulièrement avantageuse selon l'invention est une composition comprenant l'extrait de fruit peptidique et osidique de *Schizandra sphenanthera* et des peptides de riz (voir demande internationale WO 2008/009709). Cette composition est particulièrement appréciée pour ses propriétés de stimulation de la mélanogénèse et du transfert de la mélanine.

Une autre association particulièrement avantageuse selon l'invention est une composition comprenant l'extrait peptidique et osidique de fruit de *Schizandra sphenanthera* et du Cyclocéramide® (dérivé d'oxazoline) tel que décrit dans les demandes internationales WO 2004050052, WO 2004050079 et WO 2004112741. Cette composition est particulièrement adaptée pour le traitement des réactions inflammatoires.

Une autre association particulièrement avantageuse selon l'invention est une composition comprenant l'extrait peptidique et osidique de fruit de *Schizandra sphenanthera* et un extrait de Quinoa, en particulier un extrait peptidique (voir la demande internationale WO 2008/080974). Cette composition est particulièrement adaptée pour le traitement des conditions inflammatoires et de la réparation de la barrière cutanée.

Une autre association particulièrement avantageuse selon l'invention est une composition comprenant l'extrait peptidique et osidique de fruit de *Schizandra sphenanthera* et de beurre de Cupuaçu. Cette composition est particulièrement appréciée pour ses propriétés hydratantes.

Une autre association particulièrement avantageuse selon l'invention est une composition comprenant l'extrait peptidique et osidique de fruit de *Schizandra sphenanthera* et un Oléodistillat de Colza.

Une autre association avantageuse selon l'invention est une composition comprenant l'extrait peptidique et osidique de fruit de *Schizandra sphenanthera* et un Oléodistillat de Maïs.

Toutes ces associations comprennent au moins un autre composé actif, en plus de l'extrait de fruit de *Schizandra sphenanthera,* et peuvent comprendre deux, trois, quatre ou plus de composés actifs tels que décrits précédemment.

Outre ces actifs, l'extrait peptidique et osidique de fruit de *Schizandra sphenanthera* selon l'invention, seul ou en association avec les actifs précédemment cités, peut être utilisé en association des actifs protecteurs solaires, tels que des filtres ou écrans solaires UVB et/ou UVA ; tels les écrans ou filtres minéraux et/ou organiques connus de l'homme du métier qui adaptera leur choix et leurs concentrations en fonction du degré de protection recherché.

A titre d'exemple d'actifs protecteur solaire, on peut notamment citer le dioxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S), l'octocrylène, le butyl méthoxydibenzoylméthane, l'acide terephthalylidene dicamphor sulfonique, le 4-méthylbenzylidène de camphre, la benzophénone, l'éthylhexyl méthoxycinnamate, l'éthylhexyl diméthyl PABA, le diéthylhexyl butamido triazone.

La composition selon l'invention peut être formulée sous la forme de différentes préparations adaptées à une administration topique ou à une administration orale.

Selon une première variante, les différentes préparations sont adaptées à l'administration topique et incluent les crèmes, les gels, les émulsions, les laits, les pommades, les lotions, les huiles, les solutions aqueuses ou hydro-alcooliques ou glycoliques, les poudres, les patchs, les sprays ou tout autre produit pour application externe. De telles formulations sont présentées dans les exemples ci-après.

Selon une deuxième variante, les différentes préparations sont adaptées à une administration orale ; l'extrait de fruit de *Schizandra* pouvant entrer soit dans un complément alimentaire soit dans une composition alimentaire.

Le complément alimentaire peut se présenter sous forme de gélules ou de capsules molles de gélatine ou végétales dans le cadre de la présente invention. Ledit complément alimentaire peut alors contenir de 10 à 100% en poids de l'extrait peptidique de fruit de *Schizandra.*

On peut aussi incorporer, sans aucune restriction, les extraits peptidiques de fruit de *Schizandra* de la présente invention dans une composition alimentaire telle que de la nourriture, une boisson et un nutraceutique, y compris dans ceux cités ci-dessous:

1) Les produits laitiers : tels que les fromages, le beurre, le lait et autres breuvages lactés, mélanges et pâtes à tartiner à base de produits lactés, crèmes glacées et yaourts ;

2) Les produits à base de graisse tels que les margarines, pâtes à tartiner, mayonnaises, matières grasses pour cuisson, huiles à frire et vinaigrettes ;

3) Les produits à base de céréales composés de graines tels que le pain et les pâtes, que ces aliments soient cuisinés, cuits au four ou transformés.

4) Les confiseries tels que le chocolat, les bonbons, les chewing gums, les desserts, les nappages, les sorbets, les glaçages, et autres garnitures ;

5) Les boissons alcoolisées ou non, y compris les sodas et autres boissons non alcoolisées, jus de fruits, compléments diététiques, substituts de repas sous forme de breuvage comme ceux vendus sous la marque Boost™ and Ensure™ et ;

6) Les produits divers comme les oeufs, les aliments transformés comme les soupes, les sauces toute prête pour pâtes, des plats préparés et autre produits du même genre.

L'extrait peptidique de *Schizandra* peut être incorporé directement et sans autre modification dans la nourriture, les nutraceutiques, les produits diététiques notamment hyperprotéinés ou les breuvages et ce grâce à des techniques comme le mixage, l'infusion, l'injection, le mélange, l'absorption, le pétrissage et la pulvérisation. De telles formulations sont présentées dans les exemples ci-après.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, adapté à un patient comme par exemple à l'âge ou au poids corporel du patient, la gravité de son état général, la tolérance au traitement, les effets secondaires constatés, le type de peau.

L'invention décrit également une méthode de traitement cosmétique de l'acné, caractérisée en ce que l'on applique sur les zones de la peau affectées une composition cosmétique selon l'invention.

L'invention décrit également une méthode de traitement cosmétique de l'acné par voie orale, caractérisée en ce que l'individu affecté consomme par voie orale une composition nutraceutique selon l'invention.

La présente invention est également relative à un extrait peptidique et osidique de fruit de *Schizandra sphenanthera* pour son utilisation dans le traitement ou la prévention de l'acné.

Dans le cadre d'une utilisation cosmétique ou dermatologique, la composition sera avantageusement formulée sous la forme d'une préparation adaptée à une administration topique.

Dans le cadre d'une utilisation alimentaire, à visée nutritive ou cosmétique (« cosmet-food »), la composition sera avantageusement formulée sous la forme d'une préparation adaptée à une administration orale.

En particulier, cette composition est destinée à inhiber l'inflammation, via une action spécifique sur *P. acnes.* Les peptides de *Schizandra* ciblent particulièrement le processus inflammatoire de l'acné qui joue un rôle central dans le développement et l'aggravation de la pathologie. Ainsi, les exemples présentés ci-dessous montrent que les peptides de *Schizandra* ont plusieurs actions sur les kératinocytes :

- Un effet anti-inflammatoire : inhibition des médiateurs précoces (IL-1$\alpha$, IL-1$\beta$) et tardifs (IL8) de l'inflammation.
- Un effet inhibiteur sur l'induction des MMPs (2 et 9) par les kératinocytes
- Un effet inhibiteur sur la libération du VEGF

Les peptides de *Schizandra* ont également une action sur la bactérie *P.acnes* :

- Inhibition de l'induction de l'IL8 par *P. acnes*
- Inhibition de l'induction de la MMP-9 par *P. acnes*

**EXEMPLES**

**Exemple 1 : Effets de l'extrait peptidique de *Schizandra* dans la prévention et le traitement de l'acné**

**MATERIEL ET METHODES**

• *Modèle de kératinocytes en monocouche : étude de l'expression de l'IL-1$\alpha$, l'IL-1$\beta$, l'IL8, la MMP2, la MMP9*

Les kératinocytes ont été ensemencés en plaque 24 puits à raison de 120 000 cellules/puits. Après 24 heures d'incubation à 37°C, 5% de $CO_2$, les cellules ont été traitées par les peptides de *Schizandra* à 0,05% et 0,1% dans le milieu de culture pendant 48 heures.

• *Modèle de co-culture : kératinocytes /P. acnes : étude de l'expression de la MMP9*

Les cellules HaCat ont été cultivées à confluence en plaque polystyrène 24 puits à 37°C, 5% $CO_2$. Elles ont été ensuite traitées avec les peptides de *Schizandra* pendant 30 minutes, puis incubées ou non pendant 16 heures en présence de *P.acnes.*

A la fin de chaque traitement, les surnageants de culture ont été éliminés et les ARN totaux extraits à l'aide du kit d'extraction RNeasy MiniKit [Qiagen]. Les ARN totaux ont ensuite été dosés en minichips à l'aide du système Experion™ et du kit Experion RNA StdSens [Biorad] puis réverse-transcrits en cDNA à l'aide du kit iScript cDNA Synthesis [Biorad].

Les cDNA néo-synthétisés relatifs aux gènes d'intérêt (IL-1$\alpha$, IL-1$\beta$, IL8, MMP2, MMP9) ou aux gènes de référence ont été amplifiés sélectivement par PCR en temps réel sur iQ5 [Biorad] par la technologie SybrGreen [kit iQ SybrGreen, Biorad].

**• *Modèle de Kératinocytes en monocouche : effet sur l'IL-1 $\alpha$ et le VEGF***

Les kératinocytes ont été ensemencés en plaque 24 puits dans du milieu KGM2. Après 24 heures d'incubation, les cellules ont été pré-traitées par les peptides de *Schizandra* pendant 24 heures. Puis, les cellules ont été stimulées par ajout de PMA (Phorbol 12-myristate 13-acetate ; Sigma) à 10 $\mu$g/ml pendant 24 heures.

A la fin du traitement, les surnageants cellulaires ont été récoltés et l'IL-1$\alpha$ ainsi que le VEGF ont été dosé à l'aide de kits ELISA (R&D Systems), selon les instructions du fournisseur.

Sur les tapis cellulaires, un test au MTT (IL-1$\alpha$) ou au rouge neutre (VEGF) a été réalisé : la DO, proportionnelle à la quantité de cellules vivantes, est lue à 570 nm (MTT) ou 540 nm (rouge neutre).

Les quantités d'IL-1$\alpha$ et de VEGF sont exprimées en pg/ml/$DO_{570}$ et pg/ml/$DO_{570}$ respectivement.

**• *Modèle de co-culture kératinocytes / P.acnes : effet sur la production de l'Il-8***

Les cellules HaCat ont été cultivées à confluence en plaque polystyrène 24 puits à 37°C, 5% CO2. Elles ont été ensuite traitées ou non (contrôle positif) avec les peptides de *Schizandra* pendant 30 minutes, puis incubées pendant 16 heures en présence de *P.acnes.* La quantité d'IL-8 produite (en pg/ml) a été mesurée à l'aide d'un kit ELISA [R&D systems], selon les instructions du fournisseur.

**• *Méthode de calcul :***

La méthode de RT-PCR en temps réel permet la quantification relative du niveau d'expression d'un gène d'intérêt par rapport à celui d'un gène de référence en réponse à un traitement donné.

L'analyse quantitative des résultats est basée sur le recueil des cycles seuils (ou Ct pour threshold Cycle). Le cycle seuil correspond au point où le signal d'émission de fluorescence est statistiquement et significativement plus élevé que le bruit de fond. Le cycle seuil est directement corrélé au nombre de copies initiales de l'ADN cible.

Pour chaque échantillon, le niveau d'expression du gène d'intérêt a été normalisé par le niveau d'expression du gène de référence le plus stable. Le gène de référence le plus stable a été déterminé à l'aide de la macro GeNorm.

On calcule ainsi le $\Delta$Ct :

$$\Delta \mathrm{Ct} = \mathrm{Ct}_{\text{gène cible}} - \mathrm{Ct}_{\text{gène de référence}}$$

Les valeurs des $\Delta$Ct ont été comparées dans un test statistique (test t de Student) afin d'évaluer la significativité des résultats obtenus.

Dans une deuxième étape, la variation, en fonction du traitement, du nombre de copies du gène d'intérêt a été déterminée. On calcule ainsi le $\Delta\Delta$Ct :

$$\Delta\Delta \mathrm{Ct} = \Delta \mathrm{Ct}_{\text{contrôle}} - \Delta \mathrm{Ct}_{\text{traité}}$$

Enfin, la quantité relative (QR) est calculée : QR = $(1+E)^{\Delta\Delta Ct}$.

On considère que E (l'efficacité) est égale à 1, on a donc :

$$\mathrm{QR} = 2^{\Delta\Delta \mathrm{Ct}}$$

Pour l'étude de l'expression des gènes d'intérêts, le pourcentage d'inhibition a été calculé comme suit :

$$\frac{100 - \mathrm{QR}_{\text{cellules traitées}}}{\mathrm{QR}_{\text{cellules stimulées}}} \times 100$$

**• *Statistiques***

La significativité des résultats a été évaluée par un test T de Student : \$ p<0,05 ; * p<0,01.

**RESULTATS**

**A. Action des peptides de *Schizandra* sur les médiateurs primaires de l'inflammation : IL-1$\alpha$ et IL-1$\beta$**

1. Analyse de l'expression des gènes codants pour l'IL-1$\alpha$ et l'IL-1$\beta$

Après 48 heures de traitement, les peptides de *Schizandra* testées à 0,05% et 0,1% inhibent significativement l'expression génique de l'IL-1$\alpha$ (-78 à -97% ; tableau 1) et de l'IL-1$\beta$ (-80 à -90% ; tableau 2) dans les kératinocytes.

Tableau 1: Expression génique de l'IL-1$\alpha$ dans les Kératinocytes

| Analyse IL-1$\alpha$ | QR | % d'inhibition | p value |
|---|---|---|---|
| Contrôle | 1 | | - |
| Peptides de *Schizandra* 0,05% | 0,22 | -78 | p<0,01 |
| Peptides de *Schizandra* 0,1% | 0,08 | -97 | p<0,01 |

Tableau 2 : Expression génique de l'IL-$\beta$ dans les keratinocytes

| Analyse IL-1$\beta$ | QR | % d'inhibition | p value |
|---|---|---|---|
| Contrôle | 1 | | - |
| Peptides de *Schizandra* 0,05% | 0,2 | -81 | p<0,05 |
| Peptides de *Schizandra* 0,1% | 0,1 | -90 | p<0,05 |

2. Analyse du relargage de l'IL-1$\alpha$

L'influence des peptides de *Schizandra* sur la synthèse et la sécrétion de l'IL-1$\alpha$ par les kératinocytes a été ensuite évaluée. Les résultats sont présentés dans le tableau 3.
La stimulation des kératinocytes par le PMA pendant 24 heures induit un relargage significatif de l'IL-1$\alpha$ (+123%). Par contre, le pré-traitement par la déxamethasone à $10^{-7}$ M (molécule de référence) entraîne une inhibition totale (-100%) du relargage induit par le traitement au PMA. Ce résultat était attendu et valide le test.

Tableau 3 : Dosage de l'Il-1$\alpha$ relargué par les kératinocytes

D'autre part, le pré-traitement par les peptides de *Schizandra* pendant 24 heures permet d'inhiber la synthèse et le relargage de l'IL-1$\alpha$ induit par le PMA; une inhibition importante de l'ordre de 30% (à 0,1%)

**B. Action des peptides de *Schizandra* sur l'expression de l'IL-8 : médiateur secondaire de l'inflammation**

1. Analyse de l'expression du gène codant pour l'IL-8

Après 48 heures de traitement, les peptides de *Schizandra* testées à 0,05% et 0,1% inhibent significativement l'expression génique de l'IL-8 dans les kératinocytes (-73% à -86% d'inhibition) (Tableau 4).

Tableau 4 : Expression génique de l'IL-8 dans les kératinocytes

| | QR | % d'inhibition | p value |
|---|---|---|---|
| Contrôle | 1 | | - |
| Peptides de *Schizandra* 0,05% | 0,3 | -73 | p<0,01 |
| Peptides de *Schizandra* 0,1% | 0,1 | -86 | p<0,05 |

2. Effet sur la production de l'IL-8 par les kératinocytes stimulées par *P.acnes*

Le traitement des kératinocytes par une suspension de *P.acnes* stimule très fortement le relargage de l'IL-8, marqueur secondaire de l'inflammation (tableau 5).

Un pré-traitement de 30 minutes par les peptides de *Schizandra* permet d'inhiber le relargage de l'IL-8 par les kératinocytes. L'inhibition de la production d'IL-8 démarre à partir de 0,5% pour être totale à 2%.

Tableau 5 : Dosage de l'IL-8 dans les co-cultures kératinocytes / P.acnes

| | Moyenne IL-8 (pg/ml) | % stimulation (Témoin P.*acnes* par rapport au contrôle non traité) | % Inhibition (calculé par rapport au témoin positif *P. acnes*) |
|---|---|---|---|
| Contrôle non stimulé | 70,2 | | |
| Témoin positif: *P. acnes* | 804,21 | +1146 | |
| *P. acnes* + peptides de *Schizandra* à 0,5% | 630,95 | | -22 |
| *P. acnes* + peptides de *Schizandra* à 1% | 507,45 | | -37 |
| *P. acnes* + peptides de *Schizandra* à 5% | 3,95 | | -100 |

**C. Action des peptides de *Schizandra* sur les protéases matricielles : MMP2 et MMP9**

1. Analyse de l'expression de la MMP2

Le traitement des kératinocytes pendant 48 heures par les peptides de *Schizandra* (à 0,05%) entraîne une inhibition significative de l'expression génique de la MMP2 (-68% d'inhibition) (tableau 6).

Tableau 6 : Expression génique de La MMP2 dans les kératinocytes

| | QR | % inhibition | P value |
|---|---|---|---|
| Contrôle non stimulé | 1 | | |
| Peptides de *Schizandra* à 0,05% | 0,318 | -68 | P<0,01 |

2. Analyse de l'expression de la MMP9

Parmi les protéases matricielles impliquées dans la pathologie de l'acné, la MMP-9 joue un rôle particulièrement important. En effet, cette protéase est régulée positivement par des cytokines pro-inflammatoires mais également par *P. acnes.*

• *Modèle de Kératinocytes en monocouche*

Comme montré dans le tableau n°7, l'analyse des résultats de Q-PCR, a permis de montrer une inhibition de l'expression du gène de la MMP9 dans les kératinocytes après 48 heures de traitement par les peptides de *Schizandra* (-65% à 0,05 et -57% à 0,1%).

Tableau 7: Expression génique de la MMP9 dans les Kératinocytes

| | QR | % d'inhibition | P value |
|---|---|---|---|
| Contrôle | 1 | | |
| Peptides de *Schizandra* à 0,05% | 0,349 | -65 | P<0,01 |
| Peptides de *Schizandra* à 0,1% | 0,431 | -57 | P<0,01 |

• *Modèle de co-culture kératinocytes/P.acnés*

De la même façon, la modulation de l'expression de la MMP9 a été également évaluée dans le modèle de co-culture kératinocytes / *P. acnes* (tableau 8).

En utilisant ce modèle, un effet stimulateur de l'expression du gène de la MMP9 par la suspension bactérienne de *P. acnes* été mis en évidence (+37%)

Par contre, le pré-traitement des kératinocytes par les peptides de *Schizandra* permet de contrer cet effet stimulateur de *P. acnes* et l'inhibe également (-49% et -53% par rapport au kératinocytes traités par la suspension bactérienne)

Tableau 8 : Expression génique de La MMP9 dans les co-cultures kératinocytes/*P. acnes*

|  | QR | % inhibition | P value |
|---|---|---|---|
| Contrôle sans traitement | 1 |  |  |
| Témoin positif : *P. acnes* | 1,371 |  | P<0,05 |
| *P. acnes*+ peptides de *Schizandra* 0,5% | 0,69 | -49 | P<0,05 |
| *P. acnes*+ peptides de *Schizandra* 1% | 0,739 | -53 | P<0,05 |

## D. Action des peptides de *Schizandra* sur l'angiogenèse (VEGF)

Enfin, le potentiel effet inhibiteur des peptides de *Schizandra* sur la synthèse et le relargage du VEGF par des kératinocytes a été étudié. Les résultats sont présentés dans le tableau 9. Ainsi, la stimulation des kératinocytes par le PMA pendant 24 heures induit une libération importante et significative du VEGF (+269%). Par contre, le pré-traitement des kératinocytes par les peptides de *Schizandra* pendant 24 heures permet de contrer cet effet et l'inhibe de façon significative (-39% aux deux concentrations).

Tableau 9 : dosage du VEGF dans les Kératinocytes

|  | Moyenne VEGF (pg/ml) | % stimulation (PMA par rapport au contrôle non traité) | % Inhibition (peptides de *Schizandra* par rapport au PMA) | P value |
|---|---|---|---|---|
| Contrôle non stimulé | 208,166 |  |  |  |
| Témoin positif (PMA) | 767,941 | +369 |  | P<0,01 |
| Peptides de *Schizandra* à 0,05% | 466,797 |  | -39,2 | P<0,05 |
| Peptides de *Schizandra* à 0,1% | 465,736 |  | -39,4 | P<0,01 |

## Exemple 2 : exemples de formulations cosmétiques

Les inventeurs présentent ci-dessous plusieurs compositions pour application par voie topique, particulièrement indiquées dans l'acné.

Le terme « Peptides de *Schizandra* » désigne ci-après un extrait peptidique et osidique de *Schizandra chinensis* ou de *Schizandra sphenanthera*.

**CREME LAVANTE**

| Matière première / Nom commercial |  | % |
|---|---|---|
| EAU PURIFIEE |  | QSP 100 % |
| ARLATONE |  | De 10 à 30 % |
| COCOGLUCOSIDE |  | De 5 à 20 % |
| HYDROXYPROPYL GUAR |  | De 1 à 5 % |
| CAPRYLOYL GLYCINE |  | De 0 à 2 % |

(suite)

| Matière première / Nom commercial | | % |
|---|---|---|
| CONSERVATEURS | | De 0 à 2 % |
| PARFUM | | De 0 à 1 % |
| ACIDE CITRIQUE | | De 0 à 1 % |
| ZINC PCA | | De 0 à 1 % |
| **PEPTIDES DE SCHIZANDRA** | | De 0,01 à 5 % |

## EMULSION ANTI-ACNEIQUE SEBOREGULATRICE

| Matière première / Nom commercial | | % |
|---|---|---|
| PEG 40 STEARATE | | De 1 à 5 % |
| PEG 5 GLYCERYL STEAR | | De 1 à 5 % |
| CERESINE WAX | | De 1 à 5 % |
| MONOSTEARATE DE GLYCEROL | | De 1 à 5 % |
| STEARATE DE SORBITAN | | De 0 à 2 % |
| ALCOOL CETYLIQUE | | De 0 à 2 % |
| ALCOOL DI-MALATE | | De 5 à 20 % |
| VITAMINE E | | De 0 à 1 % |
| 5 ALPHA AVOCUTA | | De 1 à 5 % |
| **PEPTIDES DE SCHIZANDRA** | | De 0,01 à 5 % |
| BUTYLENE GLYCOL | | De 1 à 5 % |
| PIROCTOLAMINE | | De 0 à 1 % |
| CONSERVATEURS | | De 0 à 1 % |
| GLYCEROL | | De 1 à 10 % |
| GOMME XANTHANE | | De 0 à 1 % |
| ZINC PCA | | De 0 à 2 % |
| AMIDON DE RIZ | | De 1 à 5 % |
| NYLON 6 | | De 0 à 2 % |
| POLYACRYLAMIDE GEL | | De 1 à 5 % |
| VITAMINE B6 | | De 0 à 1 % |
| PARFUM | | De 0 à 1 % |
| EAU PURIFIÉE | | QSP 100 % |

## EMULSION ANTI-ACNEIQUE ANTI-INFLAMMATOIRE

| Matière première / Nom commercial | | % |
|---|---|---|
| PEG 40 STEARATE | | De 1 à 5 % |
| PEG 5 GLYCERYL STEAR | | De 1 à 5 % |
| CERESINE WAX | | De 1 à 5 % |
| MONOSTEARATE DE GLYCEROL | | De 1 à 5 % |

(suite)

| Matière première / Nom commercial | | % |
|---|---|---|
| STEARATE DE SORBITAN | | De 0 à 2 % |
| ALCOOL CETYLIQUE | | De 0 à 2 % |
| ALCOOL DI-MALATE | | De 5 à 20 % |
| VITAMINE E | | De 0 à 1 % |
| VITAMINE B3 | | De 1 à 5 % |
| EXTRAIT PEPTIDIQUE DE MACA | | De 1 à 5 % |
| **PEPTIDES DE SCHIZANDRA** | | De 0,01 à 5 % |
| BUTYLENE GLYCOL | | De 1 à 5 % |
| PIROCTOLAMINE | | De 0 à 1 % |
| CONSERVATEURS | | De 0 à 1 % |
| GLYCEROL | | De 1 à 10 % |
| GOMME XANTHANE | | De 0 à 1 % |
| ZINC PCA | | De 0 à 2 % |
| AMIDON DE RIZ | | De 1 à 5 % |
| NYLON 6 | | De 0 à 2 % |
| POLYACRYLAMIDE GEL | ' | De 1 à 5 % |
| VITAMINE B6 | | De 0 à 1 % |
| PARFUM | | De 0 à 1 % |
| EAU PURIFIÉE | | QSP 100 % |

**EMULSION ANTI-ACNEIQUE REPARATRICE**

| Matière première / Nom commercial | | % |
|---|---|---|
| PEG 40 STEARATE | | De 1 à 5 % |
| PEG 5 GLYCERYL STEAR | | De 1 à 5 % |
| CERESINE WAX | | De 1 à 5 % |
| MONOSTEARATE DE GLYCEROL | | De 1 à 5 % |
| STEARATE DE SORBITAN | | De 0 à 2 % |
| ALCOOL CETYLIQUE | | De 0 à 2 % |
| ALCOOL DI-MALATE | | De 5 à 20 % |
| VITAMINE E | | De 0 à 1 % |
| PANTHENOL | | De 0 à 5 % |
| EXTRAIT PEPTIDIQUE DE MACA | | De 0,1 à 5 % |
| **PEPTIDES DE SCHIZANDRA** | | De 0,01 à 5 % |
| BUTYLENE GLYCOL | | De 1 à 5 % |
| PIROCTOLAMINE | | De 0 à 1 % |
| CONSERVATEURS | | De 0 à 1 % |
| GLYCEROL | | De 1 à 10 % |

(suite)

| Matière première / Nom commercial | | % |
|---|---|---|
| GOMME XANTHANE | | De 0 à 1 % |
| ZINC PCA | | De 0 à 2 % |
| AMIDON DE RIZ | | De 1 à 5 % |
| NYLON 6 | | De 0 à 2 % |
| POLYACRYLAMIDE GEL | | De 1 à 5 % |
| VITAMINE B6 | | De 0 à 1 % |
| PARFUM | | De 0 à 1 % |
| EAU PURIFIÉE | | QSP 100 % |

## EMULSION ANTI-ACNEIQUE KERATOREGULATRICE

| Matière première / Nom commercial | | % |
|---|---|---|
| ISONONYL ISONONANOAT | | De 1 à 10 % |
| STEARATE D'ISOCETYLE | | De 1 à 10 % |
| PEG 40 STEARATE | | De 1 à 5 % |
| PEG 5 GLYCERYL STEAR | | De 1 à 5 % |
| CONSERVATEURS | | De 0 à 1 % |
| ALCOOL CETYLIQUE C16 C18 | | De 0 à 2 % |
| PPG/SMDI POLYMERE | | De 0 à 1 % |
| ACIDE SALICYLIQUE | | De 0 à 2 % |
| SQUALANE GEL | | De 0 à 2 % |
| DIOCTYL ETHER | | De 1 à 10 % |
| ALCOOL DI-MALATE | | De 1 à 10 % |
| EXTRAIT DE TOURNESOL | | De 1 à 10 % |
| TROMETHAMINE | | De 1 à 5 % |
| BUTYLENE GLYCOL | | De 1 à 10 % |
| CITRATE TRISODIQUE | | De 0 à 1 % |
| SCLEROTIUM GUM | | De 0 à 1 % |
| AMIDON DE RIZ | | De 1 à 10 % |
| POLYACRYLAMIDE GEL | | De 0 à 1 % |
| VITAMINE C | | De 0 à 2 % |
| GLYCINE | | De 0 à 2 % |
| PARFUM | | De 0 à 1 % |
| VITAMINE E | | De 0 à 2 % |
| ACIDE CITRIQUE | | De 0 à 1 % |
| **PEPTIDES DE SCHIZANDRA** | | De 0,01 à 5 % |
| EAU PURIFIÉE | | QSP 100 % |

**EMULSION ANTI-ACNEIQUE ANTI-AGE**

| Matière première / Nom commercial | | % |
|---|---|---|
| ISONONYL ISONONANOAT | | De 1 à 10 % |
| STEARATE D'ISOCETYLE | | De 1 à 10 % |
| PEG 40 STEARATE | | De 1 à 5 % |
| PEG 5 GLYCERYL STEAR | | De 1 à 5 % |
| CONSERVATEURS | | De 0 à 1 % |
| ALCOOL CETYLIQUE C16 C18 | | De 0 à 2 % |
| PPG/SMDI POLYMERE | | De 0 à 1 % |
| ACIDE SALICYLIQUE | | De 0 à 2 % |
| SQUALANE GEL | | De 0 à 2 % |
| DIOCTYL ETHER | | De 1 à 10 % |
| ALCOOL DI-MALATE | | De 1 à 10 % |
| EXTRAIT DE TOURNESOL | | De 1 à 10 % |
| TROMETHAMINE | | De 1 à 5 % |
| BUTYLENE GLYCOL | | De 1 à 10 % |
| CITRATE TRISODIQUE | | De 0 à 1 % |
| SCLEROTIUM GUM | | De 0 à 1 % |
| AMIDON DE RIZ | | De 1 à 10 % |
| POLYACRYLAMIDE GEL | | De 0 à 1 % |
| VITAMINE C | | De 0 à 2 % |
| GLYCINE | | De 0 à 2 % |
| PARFUM | | De 0 à 1 % |
| VITAMINE E | | De 0 à 2 % |
| ACIDE CITRIQUE | | De 0 à 1 % |
| RETINOL | | De 0 à 5 % |
| EXTRAIT PEPTIDIQUE DE MACA | | De 0,1 à 5 % |
| **PEPTIDES DE SCHIZANDRA** | | De 0,01 à 5 % |
| EAU PURIFIÉE | | QSP 100 % |

**STICK ROLL-ON ANTI-ACNEIQUE ANTI-BACTERIEN**

| Nom commercial | | % |
|---|---|---|
| EAU PURIFIÉE | | QSP 100 % |
| BUTYLENE GLYCOL | | De 1 à 5 % |
| **PEPTIDES DE SCHIZANDRA** | | De 0,01 à 5 % |
| ZINC PCA | | De 0 à 2 % |
| PEROXYDE DE BENZOYLE | | De 0 à 2 % |
| CARBOMER | | De 0 à 2 % |
| CONSERVATEURS | | De 0 à 1 % |

(suite)

| Nom commercial | | % |
|---|---|---|
| ACIDE CITRIQUE | | De 0 à 1 % |
| TROMETHAMINE | | De 0 à 1 % |

**STICK PHOTOPROTECTEUR ANTI-ACNEIQUE**

| Matière première / Nom commercial | | % |
|---|---|---|
| HUILE DE RICIN | | QSP 100% |
| ALCOOL OLEIQUE | | De 10 à 20% |
| HUILE PALMISTE | | De 10 à 20% |
| POLYGLYCERIN-3-BEEWAX | | De 10 à 20% |
| CIRE DE CANDELILLA | | De 10 à 20% |
| HECTORITE | | De 10 à 20% |
| TITANIUM DIOXYDE | | De 0 à 5% |
| **PEPTIDES DE SCHIZANDRA** | | **De 0,01 à 5 %** |
| BEURRE DE KARITE | | De 0 à 5% |
| VITAMINE E | | De 0 à 1% |

**CREME SOLAIRE SPF 50+ ANTI-ACNEIQUE**

| Matière première / Nom commercial | | % |
|---|---|---|
| EAU PURIFIÉE B4 | | QSP 100% |
| OXYDE DE TITANE | | De 10 à 20% |
| CYCLOPENTASILOXANE | | De 5 à 15% |
| OCTYL PALMITATE | | De 5 à 15% |
| C12-C15 ALKYL BENZOATE | | De 5 à 10% |
| DECYL PENTANOATE | | De 5 à 10% |
| OXYDE DE ZINC | | De 5 à 10% |
| GLYCEROL | | De 1 à 5% |
| PEG-45/DODECYL GLYCOL COPOLYMERE | | De 1 à 5% |
| **PEPTIDES DE SCHIZANDRA** | | **De 0,01 à 5 %** |
| CHLORURE DE SODIUM | | De 1 à 5% |
| DEXTRIN PALMITATE | | De 1 à 2% |
| VITAMINE E | | De 0 à 2% |
| CONSERVATEURS | | De 0 à 2% |
| HYDROXYPROPYL GUAR | | De 0 à 1% |
| ALOE VERA | | De 0 à 1% |
| LESSIVE SOUDE | | De 0 à 1% |
| EDTA 2 Na | | De 0 à 1% |
| GLUCONATE ZINC | | De 0 à 1% |

**SPRAY SOLAIRE SPF 50+ ANTI-ACNEIQUE**

| Matière première / Nom commercial | | % |
|---|---|---|
| CAPRYLO CAPRATE DE GLYCEROL | | De 5 à 20% |
| CYCLOPENTASILOXANE | | De 10 à 20% |
| DICAPRYLYL CARBONATE | | De 5 à 20% |
| TINOSORB S | | De 1 à 10% |
| OXYDE DE TITANE 100 | | De 10 à 20% |
| HECTORITE | | De 0 à 5% |
| ALPHA TOCOPHEROL | | De 0 à 2% |
| LAURYLGLUCOSIDE-GLYSTEARATE | | De 0 à 10% |
| EAU PURIFIÉE B4 | | QSP 100% |
| ACIDE CITRIQUE | | De 0 à 2% |
| PENTYLENE GLYCOL | | De 0 à 5% |
| GLYCEROL | | De 0 à 5% |
| GOMME XANTHANE | | De 0 à 2% |
| **PEPTIDES DE SCHIZANDRA** | | **De 0,01 à 5 %** |
| ALOE VERA | | De 0 à 1% |
| GLUCONATE ZINC | | De 0 à 1% |
| CONSERVATEURS | | De 0 à 2% |
| TINOSORB M | | De 1 à 10% |

**SOIN GOMMANT**

| Matière première / Nom commercial | | % |
|---|---|---|
| ARLATONE DUO | | De 5 à 20 % |
| AGENT GOMMANT | | De 1 à 10 % |
| SCLEROTIUM GUM | | De 1 à 10 % |
| CONSERVATEURS | | De 0 à 1 % |
| CAPRYLOYL GLYCINE | | De 0 à 1 % |
| SOUDE | | De 0 à 1 % |
| **PEPTIDES DE SCHIZANDRA** | | **De 0,01 à 5 %** |
| SEQUESTRANT | | De 0 à 1 % |
| ACIDE CITRIQUE | | De 0 à 1 % |
| EAU PURIFIÉE | | QSP 100 % |
| PARFUM | | De 0 à 1 % |

**Revendications**

1. Composition pour son utilisation dans le traitement ou la prévention de l'acné, comprenant un extrait peptidique et osidique de fruit de *Schizandra* et un excipient approprié, ledit extrait ne comprenant pas de lignanes et en ce que l'extrait peptidique et osidique est issu de *Schizandra sphenanthera*.

**2.** Composition pour son utilisation selon la revendication 1, **caractérisée en ce que** l'extrait peptidique et osidique est présent dans la composition à un pourcentage compris entre 0.01% et 15% en poids par rapport au poids total de la composition.

**3.** Composition pour son utilisation selon la revendication 2, **caractérisée en ce que** l'extrait peptique et osidique est présent dans la composition à un pourcentage compris entre 0.1% et 5% en poids par rapport au poids total de la composition.

**4.** Composition pour son utilisation selon l'une des revendications 1 à 3, **caractérisée en ce que** l'extrait peptique et osidique est constitué de :

   - 10 à 50 % de peptides, et
   - 10 à 60 % de sucres totaux,

les pourcentages étant exprimés par rapport au poids total dudit extrait peptidique.

**5.** Composition pour son utilisation selon l'une quelconque des revendications 1 à 4, **caractérisée en ce que** l'extrait peptidique et osidique est obtenu par un procédé comprenant les étapes successives suivantes :

   - à partir de fruit de *Schizandra,* extraction par $CO_2$ supercritique d'une huile brute et récupération d'un tourteau délipidé ;
   - dispersion en phase aqueuse dudit tourteau ;
   - traitement enzymatique dudit tourteau par un mélange enzymatique de cellulases, protéases, alpha-amylases, puis
   - centrifugation, ultrafiltration, diafiltration, nanofiltration et
   - récupération de l'extrait peptidique.

**6.** Composition pour son utilisation selon l'une des revendications précédentes, **caractérisée en ce qu'**elle comprend en outre au moins un agent anti-acnéique choisi parmi un agent séborégulateur, un agent anti-bactérien et/ou anti-fongique, un agent kératolytique et/ou un agent kératorégulateur, un agent astringent, un agent anti-inflammatoire et/ou anti-irritant, un agent antioxydant et/ou antiradicaliare, un agent cicatrisant, un agent anti-âge ou un agent hydratant.

**7.** Composition pour son utilisation selon la revendication 6, **caractérisée en ce que** l'agent anti-acnéique est choisi parmi un inhibiteur de 5 alpha réductase, l'acide pyroglutamique, l'acide linoléique, le capryloyl glycine, le zinc et ses dérivés, le cuivre, les oligoéléments, le peroxyde de benzoyle, le ketonazole, l'érythromycine, les acides de fruit alpha hydroxyacides et leurs esters ou dérivés, l'acide salicylique et ses dérivés, l'acide azélaïque, les polyphénols, l'acide glycyrrhetinique et ses esters, l'alpha bisabolol, les peptides de Lupin, l'oléodistillat de Tournesol, les sucres d'Avocat, les peptides d'Avocat, les furanes d'avocat, un dérivé d'oxazoline, l'extrait peptidique de Quinoa, l'extrait peptidique de Maca, l'acide lipoïque, le beta carotène, la vitamine B3 (niacinamide), le lycopène, la vitamine E, les anthocyanes et flavonoïdes, les isoflavones, la vitamine B5 (panthénol), la vitamine C, le coenzyme Q10, la glucosamine et ses dérivés, les glucosaminoglycanes, l'adapalène, le tazarotène, les rétinoïdes, l'acide pyrrolidone carboxylique, les céramides, l'acide hyaluronique, la glycérine et l'urée.

**8.** Extrait peptidique et osidique de fruit de *Schizandra sphenanthera,* ledit extrait ne comprenant pas de lignanes pour son utilisation dans le traitement ou la prévention de l'acné.

**9.** Extrait pour son utilisation selon la revendication 8, **caractérisé en ce que** ledit extrait est destiné à inhiber l'inflammation via une action spécifique sur *P. acnes.*

**Patentansprüche**

**1.** Zusammensetzung zur Verwendung bei der Behandlung oder der Vorbeugung von Akne, umfassend einen peptidischen und osidischen Extrakt der Frucht von *Schisandra* und einen geeigneten Trägerstoff, wobei der Extrakt keine Lignane umfasst, und dadurch, dass der peptidische und osidische Extrakt aus *Schisandra sphenanthera* stammt.

**2.** Zusammensetzung zur Verwendung nach Anspruch 1, **dadurch gekennzeichnet, dass** der peptidische und osidische Extrakt in der Zusammensetzung in einem Prozentsatz vorhanden ist, der zwischen 0,01 Gew% und 15 Gew% mit Bezug auf das Gesamtgewicht der Zusammensetzung liegt.

**3.** Zusammensetzung zur Verwendung nach Anspruch 2, **dadurch gekennzeichnet, dass** der peptidische und osidische Extrakt in der Zusammensetzung in einem Prozentsatz vorhanden ist, der zwischen 0,1 Gew% und 5 Gew% mit Bezug auf das Gesamtgewicht der Zusammensetzung liegt.

**4.** Zusammensetzung zur Verwendung nach einem der Ansprüche 1 bis 3, **dadurch gekennzeichnet, dass** der peptidische und osidische Extrakt besteht aus:

- 10 bis 50 % Peptiden und
- 10 bis 60 % Gesamtzuckern,

wobei die Prozentsätze mit Bezug auf das Gesamtgewicht des peptidischen Extrakts ausgedrückt sind.

**5.** Zusammensetzung zur Verwendung nach einem der Ansprüche 1 bis 4, **dadurch gekennzeichnet, dass** der peptidische und osidische Extrakt erhalten wird durch ein Verfahren, umfassend die folgenden sukzessiven Schritte:

- ausgehend von der Frucht von *Schisandra,* Extrahieren durch superkritisches $CO_2$ eines Rohöls und Wiedergewinnen eines entfetteten Kuchens;
- Dispergieren in wässriger Phase des Kuchens;
- enzymatisches Behandeln des Kuchens durch eine enzymtische Mischung von Zellulasen, Proteasen, Alpha-Amylasen, dann
- Zentrifugieren, Ultrafiltrieren, Diafiltrieren, Nanofiltrieren und
- Wiedergewinnen des peptidischen Extrakts.

**6.** Zusammensetzung zur Verwendung nach einem der vorhergehenden Ansprüche, **dadurch gekennzeichnet, dass** sie außerdem mindestens ein Anti-Akne-Mittel umfasst, ausgewählt aus einem Sebumregulierer, einem antibakteriellen und/oder antimykotischen Mittel, einem antikeratolytischen und/oder einem keratoregulierenden Mittel, einem astringenten Mittel, einem entzündungshemmenden und/oder reizvermeidenden Mittel, einem Antioxidations- und/oder antiradikalischen Mittel, einem Vernarbungsmittel, einem Anti-Aging-Mittel oder einem Feuchthaltemittel.

**7.** Zusammensetzung zur Verwendung nach Anspruch 6, **dadurch gekennzeichnet, dass** das Anti-Akne-Mittel ausgewählt ist aus einem Hemmer von 5-Alpha-Reduktase, Pyroglutaminsäure, Linolsäure, Capryloylglycin, Zink und seinen Derivaten, Kupfer, Oligoelementen, Benzoylperoxyd, Ketonazol, Erythromycin, Alpha-Hydroxy-Fruchtsäuren und ihren Estern und Derivaten, Salicylsäure und ihren Derivaten, Azelainsäure, Polyphenole, Glycyrrhetinsäure und ihren Estern, Alpha-Bisabolol, Peptide der Lupine, Öldestillat von Sonnenblumen, Zucker von Avocado, Peptiden von Avocado, Furanen von Avocado, Derivaten von Oxazolin, Peptidextrakt von Quinoa, Peptidextrakt von Maca, Liponsäure, Betacarotin, Vitamin B3 (Niacinamid), Lycopin, Vitamin E, Anthocyanen und Flavonoiden, Isoflavonen, Vitamin B5 (Panthenol), Vitamin C, Coenzym Q10, Glucosamin und seinen Derivaten, Glucosaminoglykanen, Adapalen, Tazaroten, Retinoiden, Pyrrolidoncarbonsäure, Ceramiden, Hyaluronsäure, Glycerin und Harnstoff.

**8.** Peptidischer und osidischer Extrakt der Frucht von *Schisandra sphenanthera,* wobei der Extrakt keine Lignane zur Verwendung bei der Behandlung oder der Vorbeugung von Akne umfasst.

**9.** Extrakt zur Verwendung nach Anspruch 8, **dadurch gekennzeichnet, dass** der Extrakt dazu ausgelegt ist, um die Entzündung über eine spezifische Wirkung auf *P. acnes* zu hemmen.

**Claims**

**1.** Composition for use in the treatment or prevention of acne, comprising a peptide and sugar extract of *Schizandra* fruit and a suitable excipient, said extract not comprising any lignans, and in that the peptide and sugar extract comes from *Schizandra sphenanthera.*

**2.** Composition for use according to claim 1, **characterised in that** the peptide and sugar extract is present in the composition in a proportion of between 0.01% and 15% by weight with respect to the total weight of the composition.

3. Composition for use according to claim 2, **characterised in that** the peptide and sugar extract is present in the composition in a proportion of between 0.1% and 5% by weight with respect to the total weight of the composition.

4. Composition for use according to any of claims 1 to 3, **characterised in that** the peptide and sugar extract consists of:

    - 10% to 50% peptides, and
    - 10% to 60% of total sugars,

the percentages being expressed with respect to the total weight of said peptide extract.

5. Composition for use according to any of claims 1 to 4, **characterised in that** the peptide and sugar extract is obtained by a method comprising the following successive steps:

    - from *Schizandra* fruit, extraction of a crude oil by supercritical $CO_2$ and recovery of defatted oilcake;
    - dispersion of said oilcake in aqueous phase;
    - enzymatic treatment of said oilcake by an enzymatic mixture of cellulases, proteases and alpha-amylases, and then
    - centrifugation, ultrafiltration, diafiltration, nanofiltration, and
    - recovery of the peptide extract.

6. Composition for use according to any of the preceding claims, **characterised in that** it also comprises at least one anti-acne agent chosen from a seboregulator agent, an anti-bacterial and/or antifungal agent, a keratolytic agent and/or a keratoregulator agent, an astringent agent, an antiinflammatory and/or anti-irritant agent, an antioxidant and/or antiradical agent, a healing agent, an anti-age agent or a moisturiser.

7. Composition for use according to claim 6, **characterised in that** the anti-acne agent is chosen from a 5 alpha reductase inhibiter, pyroglutamic acid, linoleic acid, glycine capryloyl, zinc and derivatives thereof, copper, oligoelements, benzoyl peroxide, ketonazole, erythromycin, alpha-hydroxyacid fruit acids and esters or derivatives thereof, salicylic acid and derivatives thereof, azelaic acid, polyphenols, glycyrrhetinic acid and esters thereof, alpha bisabolol, lupin peptides, sunflower oleodistillate, avocado sugars, avocado peptides, avocado furans, an oxazoline derivative, quinoa peptide extract, maca peptide extract, lipoic acid, beta carotene, vitamin B3 (niacinamide), lycopene, vitamin E, anthocyans and flavonoids, isoflavones, vitamin B5 (panthenol), vitamin C, coenzyme Q10, glucosamine and derivatives thereof, glucosaminoglycans, adapalene, tazarotene, retinoids, pyrrolidone carboxylic acid, ceramides, hyaluronic acid, glycerin and urea.

8. Peptide and sugar extract of *Schizandra sphenanthera* fruit, said extract not comprising any lignans, for use in the treatment or prevention of acne.

9. Extract for use according to claim 8, **characterised in that** said extract is intended to inhibit inflammation via a specific action on *P. acnes.*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20030026854 A, Mr Zhao
- WO 2006122485 A
- WO 20061222454 A
- WO 2007020382 A
- WO 2007005760 A
- KR 2001931
- CN 11040971
- WO 2005115421 A
- WO 2005105123 A
- WO 2004012496 A
- WO 2004012752 A
- WO 2004016106 A
- WO 2007057439 A
- WO 0121605 A
- WO 0121150 A
- WO 0151596 A
- FR 2822821
- FR 2857596
- WO 2005102259 A
- WO 9847479 A
- WO 2004112742 A
- WO 2008009709 A
- WO 2004050052 A
- WO 2004050079 A
- WO 2004112741 A
- WO 2008080974 A